(19) Europäisches Patentamt
European Patent Office
Office européen des brevets

(11) Numéro de publication: **0 338 892**
**A1**

(12) **DEMANDE DE BREVET EUROPEEN**

(21) Numéro de dépôt: **89401009.9**

(22) Date de dépôt: **12.04.89**

(51) Int. Cl.[4]: **C 07 D 281/10**
C 07 D 417/12, A 61 K 31/55

(30) Priorité: **19.04.88 FR 8805131**

(43) Date de publication de la demande:
**25.10.89 Bulletin 89/43**

(84) Etats contractants désignés:
**AT BE CH DE ES FR GB GR IT LI LU NL SE**

(71) Demandeur: **SYNTHELABO**
**58 rue de la Glacière**
**F-75013 Paris (FR)**

(72) Inventeur: **Lochead, Alistair**
**142, Rue Saint Maur**
**F-75011 Paris (FR)**

**Muller, Jean Claude**
**4, Avenue de l'Espérance**
**F-91390 Morsang sur Orge (FR)**

**Denys, Colombe**
**7, Rue André Theuriet**
**F-92340 Bourg la Reine (FR)**

**Dumas, André**
**9, Clos Madeleine**
**F-91120 Palaiseau (FR)**

(74) Mandataire: **Ludwig, Jacques et al**
**SYNTHELABO Service Brevets 58, rue de la Glacière**
**F-75013 Paris (FR)**

Revendications pour les Etats contractants suivants: ES + GR.

(54) **Dérivés de [[N-[amino-2 oxo-2 éthyl] méthylamino]-2 éthyl]-5 hydroxy-3 (méthoxy-4 phényl)-2 dihydro-2,3 5H-benzothiazépine-1,5 one-4, leur préparation et leur application en thérapeutique.**

(57) Composés, sous forme de diastéréoisomères purs ou de leurs mélanges, répondant à la formule générale (I)

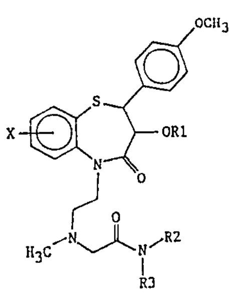

(I)

dans laquelle R1 représente un atome d'hydrogène ou un groupe alcanoyle en $C_2$-$C_4$, R2 et R3, indépendamment l'un de l'autre, représentent chacun un atome d'hydrogène, un groupe alkyle en $C_1$-$C_4$ linéaire ou ramifié, un groupe cycloalkyle en $C_3$-$C_5$, un groupe phényle, un groupe benzyle ou un groupe phénéthyle, ou bien encore R2 et R3 forment ensemble, avec l'atome d'azote qui les porte, un hétérocycle choisi parmi les groupes pyrrolidinyle substitué ou non, pipéridinyle substitué ou non, tétrahydro-1,2,3,6 pyridinyle, perhydroazépinyle, perhydroazocinyle, thiazolidine-1,3 yle, thiomorpholinyle, morpholinyle, dihydro-2,3 indolyle, tétrahydro-1,2,3,4 isoquinoléinyle substitué ou non, ou tétrahydro-2,3,4,5 1H-benzo[b] azépinyle, et X représente un atome d'hydrogène ou de chlore. Application en thérapeutique.

EP 0 338 892 A1

## Description

### DERIVES DE [[N-[AMINO-2 OXO-2 ETHYL]METHYLAMINO]-2 ETHYL]-5 HYDROXY-3 (METHOXY-4 PHENYL)-2 DIHYDRO-2,3 5H-BENZOTHIAZEPINE-1,5 ONE-4, LEUR PREPARATION ET LEUR APPLICATION EN THERAPEUTIQUE

La présente invention a pour objet des dérivés de [[N-[amino-2 oxo-2 éthyl]méthylamino]-2 éthyl]-5 hydroxy-3 (méthoxy-4 phényl)-2 dihydro-2,3 5H-benzothiazépine-1,5 one-4, leur préparation et leur application en thérapeutique.

Les composés de l'invention répondent à la formule générale (I) donnée dans le schéma ci-après, formule dans laquelle

R1 représente un atome d'hydrogène ou un groupe alcanoyle en $C_2$-$C_4$,

R2 et R3, indépendamment l'un de l'autre, représentent chacun un atome d'hydrogène, un groupe alkyle en $C_1$-$C_4$ linéaire ou ramifié, un groupe cycloalkyle en $C_3$-$C_5$, un groupe phényle, un groupe benzyle ou un groupe phénéthyle, ou bien encore R2 et R3 forment ensemble, et avec l'atome d'azote qui les porte, un hétérocycle choisi parmi les groupes pyrrolidinyle, diméthyl-2,2 pyrrolidinyle, pipéridinyle, phényl-4 pipéridinyle, (méthoxy-3 phényl)-4 pipéridinyle, (fluoro-4 benzoyl)-4 pipéridinyle, tétrahydro-1,2,3,6 pyridinyle, perhydroazépinyle, perhydroazocinyle, thiazolidine-1,3 yle, thiomorpholinyle, morpholinyle, dihydro-2,3 indolyle, tétrahydro-1,2,3,4 isoquinoléinyle, méthoxy-6 tétrahydro-1,2,3,4 isoquinoléinyle, diméthoxy-6,7 tétrahydro-1,2,3,4 isoquinoléinyle ou tétrahydro-2,3,4,5 1H-benzo[b]azépinyle, et

X représente un atome d'hydrogène ou de chlore.

Parmi les composés dans la formule générale (I) desquels R2 et R3 sont deux substituants séparés, on préfère ceux où R3 représente un groupe benzyle (et en particulier la acétyloxy-3 (méthoxy-4 phényl)-2 [[N-[oxo-2 [N'-(phénylméthyl)méthylamino]-2 éthyl]méthylamino]-2 éthyl]-5 dihydro-2,3 5H-benzothiazépine-1,5 one-4).

Parmi les composés dans la formule générale (I) desquels R2 et R3 sont réunis, on préfère ceux où R2 et R3 représentent, avec l'atome d'azote qui les porte, soit un groupe phényl-4 pipéridinyle (et en particulier la acétyloxy-3 (méthoxy-4 phényl)-2 [[N-[oxo-2 (phényl-4 pipéridinyl-1)-2 éthyl]méthylamino]-2 éthyl]-5 dihydro-2,3 5H-benzothiazépine-1,5 one-4), soit un groupe perhydroazépinyle (et en particulier la acétyloxy-3 (méthoxy-4 phényl)-2 [[N-[oxo-2 (perhydroazépi

SCHEMA

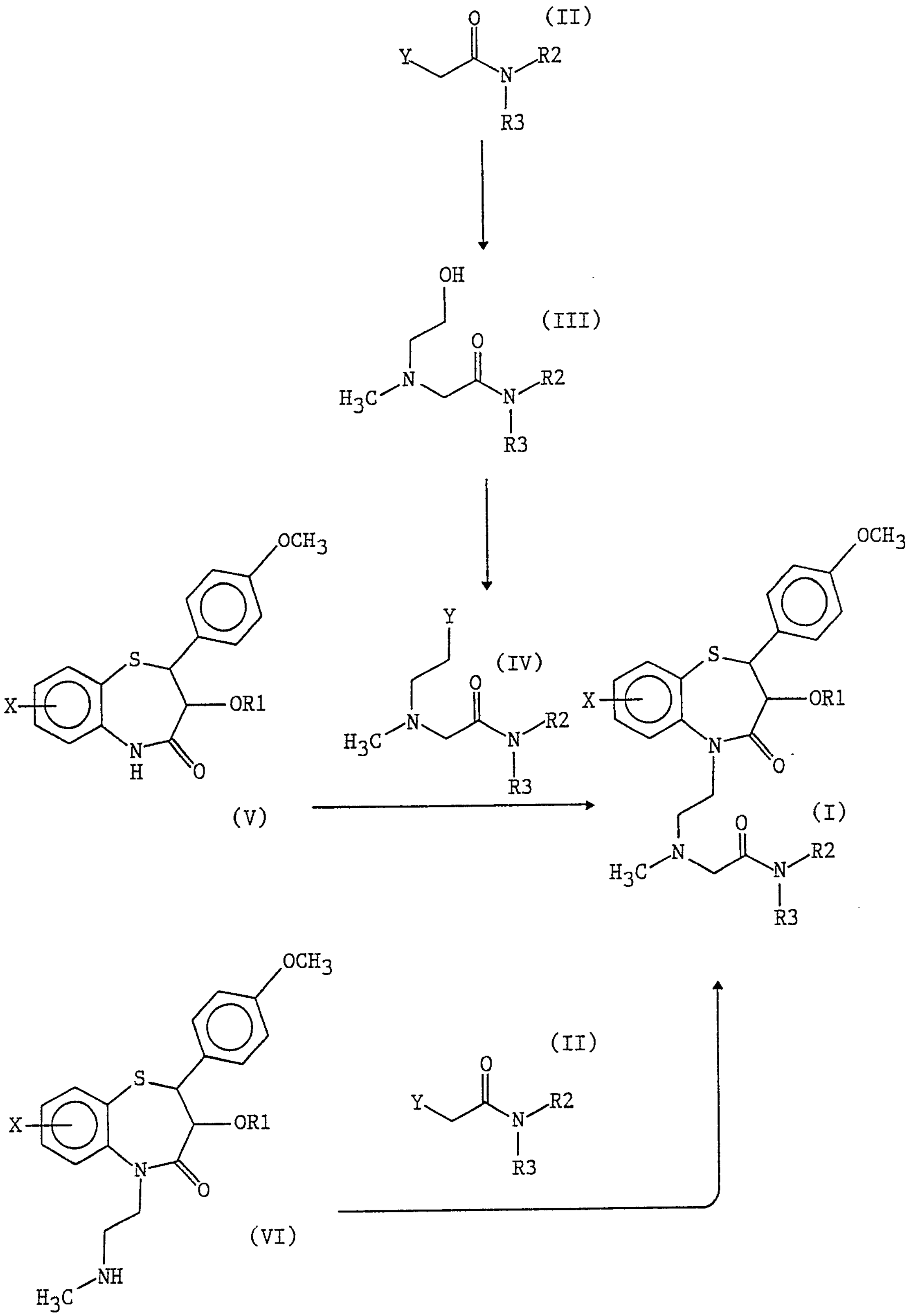

(II)

(III)

(IV)

(V)

(I)

(II)

(VI)

nyl-1)-2 éthyl]méthylamino]-2 éthyl]-5 dihydro-2,3 5H-benzothiazépine-1,5 one-4, la chloro-9 hydroxy-3

(méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4 et la acétyloxy-3 chloro-9 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4), soit encore un groupe tétrahydro-1,2,3,4 isoquinoléinyle éventuellement substitué (et en particulier la acétyloxy-3 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4, la acétyloxy-3 (méthoxy-4 phényl)-2 [[N-[(méthoxy-6 tétrahydro-1,2,3,4 isoquinoléinyl-2)-2 oxo-2 éthyl]méthylamino]-2 éthyl]-5 dihydro-2,3 5H-benzothiazépine-1,5 one-4, la chloro-9 hydroxy-3 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4 et la acétyloxy-3 chloro-9 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4.

Les composés de formule générale (I) peuvent se présenter à l'état de bases libres ou de sels d'addition à des acides.

Les atomes de carbone aux positions 2 et 3 étant asymétriques, ils peuvent aussi exister sous diverses formes diastéréoisomères, racémiques ou optiquement pures, la forme préférée étant la forme (+)-cis (2S,3S). Ces diverses formes font partie de l'invention.

Conformément à l'invention on peut préparer les composés de formule générale (I) selon le schéma donné ci-avant.

Une première méthode consiste à faire réagir une benzothiazépinone de formule (V) (dans laquelle X et R1 sont tels que définis ci-dessus) avec une amine halogénée de formule (IV) (dans laquelle R2 et R3 sont tels que définis ci-dessus et Y est un atome d'halogène tel que le chlore ou le brome).

La seconde méthode consiste à faire réagir une benzothiazépinone de formule (VI) (dans laquelle X et R1 sont tels que définis ci-dessus) avec un dérivé halogéné de formule (III) (dans laquelle R2 et R3 sont tels que définis ci-dessus et Y est un atome d'halogène tel que le chlore ou le brome).

Ces deux réactions, entre une amine et un dérivé halogéné, sont classiques, et se déroulent donc dans des conditions bien connues de l'homme du métier, c'est-à-dire par exemple dans un solvant aprotique, tel que l'acétone ou la butanone-2, à la température du reflux, en présence d'une base destinée à fixer l'acide libéré, par exemple le carbonate de potassium, et éventuellement en présence d'un catalyseur de transfert de phase tel que l'iodure de tétra-n-butylammonium.

Il va de soi que l'on peut transformer un composé de formule (I) dans laquelle R1 représente un atome d'hydrogène en un composé de formule (I) dans laquelle R1 représente un groupe alcanoyle par une acylation de type connu, et que la transformation inverse est possible par hydrolyse.

Les benzothiazépinones de formule (V) sont décrites dans le brevet des Etats-Unis d'Amérique N°3562257 ; celles de formule (VI) sont décrites dans les demandes de brevets européens N°158339 et 158340.

L'amine halogénée de formule (IV) peut être préparée par exemple à partir de l'alcool de formule (III), par action d'un agent d'halogénation tel que le chlorure de thionyle.

L'aminoalcool de formule (III) peut lui même être préparé selon toute méthode connue, par exemple à partir d'un dérivé halogéné de formule (II) et de méthylamino-2 éthanol.

Les composés de formule (II), décrits dans la littérature, sont accessibles à partir de chlorure de chloroacétyle et d'une amine de formule HNR2R3 (dans laquelle R2 et R3 sont tels que définis ci-dessus).

Les exemples qui vont suivre illustrent en détail la préparation de quelques composés conformes à l'invention. Les microanalyses élémentaires et les spectres IR et RMN confirment les structures des produits obtenus.

Les numéros indiqués entre parenthèses pour chaque exemple correspondent à ceux du tableau donné plus loin.

Exemple 1 (Composé n°17)

(+)-cis-(2S,3S)-acétyloxy-3 (méthoxy-4 phényl)-2 [[N-[oxo-2 (phényl-4 pipéridinyl-1)-2 éthyl]méthylamino]-2 éthyl]-5 dihydro-2,3 5H-benzothiazépine-1,5 one-4, oxalate.

On chauffe au reflux pendant 8 h un mélange de 3 g (6,9 mmoles) de (+)-cis-(2S,3S)-acétyloxy-3 (méthoxy-4 phényl)-2 (méthylamino-2 éthyl)-5 dihydro-2,3 5H-benzothiazépine-1,5 one-4 sous forme de chlorhydrate, 1,96 g (8,2 mmoles) de (chloro-2 acétyl)-1 phényl-4 pipéridine et 4 g (29 mmoles) de carbonate de potassium dans 80 ml de butanone-2.

On filtre le mélange, on évapore le filtrat et on purifie le résidu par chromatographie sur gel de silice. On reprend l'huile obtenue avec de l'éthanol, on la traite avec de l'acide oxalique, on filtre le précipité et on le recristallise dans l'éthanol. On isole 2,2 g d'oxalate.

Point de fusion : 167°C $[\alpha]_D^{20} = +69,1°$ (c = 0,5 ; MeOH).

Exemple 2 (Composé n°28)

(+)-cis-(2S,3S)-acétyloxy-3 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4, oxalate.

On chauffe au reflux pendant 10 h un mélange de 10 g (23 mmoles) de (+)-cis-(2S,3S)-acétyloxy-3 (méthoxy-4 phényl)-2 (méthylamino-2 éthyl)-5 dihydro-2,3 5H-benzothiazépine-1,5 one-4 sous forme de chlorhydrate, 5,28 g (25 mmoles) de (chloro-2 acétyl)-2 tétrahydro-1,2,3,4 isoquinoléine et 13 g de carbonate

de potassium dans 200 ml de butanone-2.
On filtre le mélange, on évapore le filtrat, on reprend le résidu avec 80 ml de chloroforme et on le lave avec 50 ml d'eau. On sèche la phase organique sur sulfate de magnésium, on l'évapore et on purifie l'huile résiduelle par chromatographie sur gel de silice.
On obtient 10,1 g de base libre qu'on reprend dans de l'éthanol et qu'on traite avec de l'acide oxalique. On filtre le précipité et on le recristallise dans l'éthanol.
On isole 8,03 g d'oxalate.
Point de fusion : 144°C $[\alpha]_D^{20}$ = +77,5° (c = 0,5 ; MeOH).

Exemple 3 (Composé n°29).

(+)-cis-(2S,3S)-acétyloxy-3 (méthoxy-4 phényl)-2 [[N-[(méthoxy-6 tétrahydro-1,2,3,4 isoquinoléinyl-2)-2 oxo-2 éthyl]méthylamino]-2 éthyl]-5 dihydro-2,3 5H-benzothiazépine-1,5 one-4, oxalate.
On chauffe au reflux pendant 20 h un mélange de 3,77 g (8,6 mmoles) de (+)-cis-(2S,3S)-acétyloxy-3 (méthoxy-4 phényl)-2 (méthylamino-2 éthyl)-5 dihydro-2,3 5H-benzothiazépine-1,5 one-4 sous forme de chlorhydrate, 2,13 g (9,5 mmoles) de (chloro-2 acétyl)-2 méthoxy-6 tétrahydro-1,2,3,4 isoquinoléine et 5,2 g (38 mmoles) de carbonate de potassium dans 100 ml de butanone-2.
On filtre le mélange, on évapore le filtrat, on reprend le résidu avec 80 ml de chloroforme et on le lave avec 50 ml d'eau. On sèche la phase organique sur sulfate de magnésium, on l'évapore et on purifie l'huile résiduelle par chromatographie sur gel de silice.
On obtient 4,19 g de base libre qu'on reprend dans de l'éthanol et qu'on traite avec de l'acide oxalique. On filtre le précipité et on le recristallise dans l'éthanol.
On isole 1,93 g d'oxalate.
Point de fusion : 142°C $[\alpha]_D^{20}$ = +73,3° (c = 0,5 ; MeOH).

Exemple 4 (Composé n°21).

(+)-cis-(2S,3S)-acétyloxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, oxalate.
On chauffe au reflux pendant 15 h un mélange de 10 g (22 mmoles) de (+)-cis-(2S,3S)-acétyloxy-3 (méthoxy-4 phényl)-2 (méthylamino-2 éthyl)-5 dihydro-2,3 5H-benzothiazépine-1,5 one-4 sous forme de chlorhydrate, 5,2 g (29 mmoles) de (chloro-2 acétyl)-1 perhydroazépine et 12,67 g (92 mmoles) de carbonate de potassium dans 200 ml de butanone-2.
On filtre le mélange, on évapore le filtrat, on reprend le résidu avec 50 ml de chloroforme et on le lave avec 30 ml d'eau. On sèche la phase organique sur sulfate de magnésium, on l'évapore et on purifie l'huile résiduelle par chromato graphie sur gel de silice.
On reprend la base purifiée avec de l'éthanol, on la traite avec 1,46 g (16 mmoles) d'acide oxalique, on filtre le précipité et on le recristallise dans l'éthanol.
On isole 8,21 g d'oxalate.
Point de fusion : 147-149°C $[\alpha]_D^{20}$ = +86,11° (c = 0,5 ; MeOH).

Exemple 5 (Composé n°20).

(+)-cis-(2S,3S)-hydroxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, oxalate.
On refroidit à 0°C une solution de 6,53 g (12 mmoles) de (+)-cis-(2S,3S)-acétyloxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4 dans 100 ml de méthanol, on ajoute 0,5 ml d'une solution 6N de méthylate de sodium, et on agite le mélange pendant 4 h. On évapore le solvant, on reprend le résidu avec du chloroforme et on le lave successivement avec de l'eau bicarbonatée puis avec de l'eau. On sèche la phase organique et on l'évapore. On reprend l'huile résiduelle avec 40 ml d'éthanol, on ajoute 0,934 g d'acide oxalique et on recristallise le produit obtenu dans un mélange 1/2 d'acétone/alcool isopropylique.
On isole finalement 4,54 g d'oxalate.
Point de fusion : 102°C $[\alpha]_D^{20}$ = +82,3° (c = 0,5 ; MeOH).

Exemple 6 (Composé n°37).

(+)-cis-(2S,3S)-chloro-9 hydroxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, oxalate.

a) [N-[oxo-2 (perhydroazépinyl-1)-2 éthyl]méthylamino]-2 éthanol.
On chauffe à 70°C pendant 5 h un mélange de 4,8 g (27 mmoles) de (chloro-2 acétyl)-1 perhydroazépine en solution dans 40 ml de butanone-2, 3,6 g (48 mmoles) de méthylamino-2 éthanol et 6,6 g (48 mmoles) de carbonate de potassium.
On filtre le mélange à chaud, on évapore le filtrat, on le reprend avec du dichlorométhane, on lave la solution

avec de la soude 1N, on la sèche sur sulfate de magnésium et on l'évapore. On obtient 5,7 g de produit qu'on utilise tel quel dans l'étape suivante.

b) Chloro-2 N-méthyl-N-[oxo-2 (perhydroazépinyl-1)-2 éthyl]-éthylamine.

On chauffe au reflux pendant 2 h un mélange de 5,7 g (27 mmoles) de [N-[oxo-2 (perhydroazépinyl-1)-2 éthyl]méthylamino]-2 éthanol en solution dans 50 ml de chloroforme et 6 ml (excès) de chlorure de thionyle. Ensuite on évapore le mélange, on reprend le résidu avec du benzène, on l'évapore et on reprend le résidu avec de l'éther. On lave la solution avec une solution froide de soude 2N, on sépare l'insoluble par filtration, on sèche le filtrat sur sulfate de magnésium et on l'évapore.

On obtient 5 g de produit qu'on utilise tel quel dans l'étape suivante.

c) (+)-cis-(2S,3S)-chloro-9 hydroxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, oxalate.

On chauffe au reflux pendant 18 h une suspension de 4 g (12 mmoles) de (+)-cis-(2S,3S)-chloro-9 hydroxy-3 (méthoxy-4 phényl)-2 dihydro-2,3 5H-benzothiazépine-1,5 one-4, 3 g (13 mmoles) de chloro-2 N-méthyl-N-[oxo-2 (perhydroazépinyl-1)-2 éthyl]-éthylamine et 5,5 g (39 mmoles) de carbonate de potassium dans 60 ml de butanone-2.

On filtre l'insoluble, on évapore le filtrat, on reprend le résidu avec du dichlorométhane, on lave la solution avec de l'eau bicarbonatée, on la sèche sur sulfate de magnésium et on l'évapore. On traite le résidu avec un équivalent d'acide oxalique, et on obtient 5,5 g d'oxalate.

Point de fusion : 108°C $[\alpha]_D^{20} = +21°$ (c = 1 ; MeOH).

Exemple 7 (Composé n°39).

(+)-cis-(2S,3S)-acétyloxy-3 chloro-9 méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, chlorhydrate.

On chauffe à 100°C pendant 6 h un mélange de 3,55 g (7 mmoles) de (+)-cis-(2S,3S)-chloro-9 hydroxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, (base libre), 30 ml d'acide acétique, 30 ml d'anhydride acétique et 3,5 ml d'éther saturé de chlorure d'hydrogène.

On évapore le mélange, on reprend le résidu avec du toluène, on évapore ce dernier et on triture le résidu dans un mélange d'acétate d'éthyle et d'éther.

On isole finalement 3,47 g de chlorhydrate.

Point de fusion : 126°C $[\alpha]_D^{20} = +21,1°$ (c = 1 ; MeOH).

Exemple 8 (Composé n°36).

(±)-cis-(2S,3S)-chloro-9 hydroxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, oxalate.

On procède de manière analogue à celle décrite dans l'exemple 6c), en chauffant à 70°C pendant 20 h un mélange de 3,35 g (10 mmoles) de (±)-cis-(2S,3S)-chloro-9 hydroxy-3 (méthoxy-4 phényl)-2 dihydro-2,3 5H-benzothiazépine-1,5 one-4, 2,5 g (11 mmoles) de chloro-2 N-méthyl-N-[oxo-2 (perhydroazépinyl-1)-2 éthyl]-éthylamine, et 4,7 g (30 mmoles) de carbonate de potassium dans 60 ml de butanone-2.

On isole finalement 4,6 g d'oxalate.

Point de fusion : 118°C.

Exemple 9 (Composé n°38).

(±)-cis-(2S,3S)-acétyloxy-3 chloro-9 méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, chlorhydrate.

L'acétylation de 1,7 g de (±)-cis-(2S,3S)-chloro-9 hydroxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, oxalate dans des conditions analogues à celles de l'exemple 7 permet d'obtenir 1,7 g de chlorhydrate.

Point de fusion : 148°C.

Exemple 10 (Composé n°33).

(+)-cis-(2S,3S)-chloro-9 hydroxy-3 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4, chlorhydrate.

a) [N-[oxo-2 (tétrahydro-1,2,3,4 isoquinoléinyl-2)-2 éthyl]méthylamino]-2 éthanol.

On procède de manière analogue à celle décrite dans l'exemple 6a), en faisant réagir 4,78 g (23 mmoles) de (chloro-2 acétyl)-2 tétrahydro-1,2,3,4 isoquinoléine et 3 g (40 mmoles) de méthylamino-2 éthanol.

On obtient 5,6 g de produit.

b) Chloro-2 N-méthyl-N-[oxo-2 (tétrahydro-1,2,3,4 isoquinoléinyl-2)-2 éthyl]-éthylamine.

On procède de manière analogue à celle décrite dans l'exemple 6b) en traitant les 5,6 g de [N-[oxo-2 (tétrahydro-1,2,3,4 isoquinoléinyl-2)-2 éthyl]méthylamino]-2 éthanol avec un excès de chlorure de thionyle dans le chloroforme.

On obtient 5,2 g de produit.

c) (+)-cis-(2S,3S)-chloro-9 hydroxy-3 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4, chlorhydrate.

On procède de manière analogue à celle décrite dans l'exemple 6c) en faisant réagir 5 g (15 mmoles) de (+)-cis-(2S,3S)--chloro-9 hydroxy-3 (méthoxy-4 phényl)-2 dihydro-2,3 5H-benzothiazépine-1,5 one-4 et 4,2 g (15,7 mmoles) de chloro-2 N-méthyl-n-[oxo-2 (tétrahydro-1,2,3,4 isoquinoléinyl-2)-2 éthyl]-éthylamine dans la butanone-2, en présence de carbonate de potassium.

On obtient 5,45 g de produit brut dont on prépare le chlorhydrate au moyen d'éther saturé de chlorure d'hydrogène.

On obtient finalement 4,4 g de chlorhydrate.

Point de fusion : 162°C $[\alpha]_D^{20}$ = 20,3° (c = 1 ; MeOH).

Exemple 11 (Composé n°35).

(+)-cis-(2S,3S)-acétyloxy-3 chloro-9 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4, chlorhydrate.

L'acétylation du (+)-cis-(2S,3S)-chloro-9 hydroxy-3 (méthoxy-4 phényl)-2[[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4, dans des conditions analogues à celles décrites dans l'exemple 7 permet d'obtenir quantitativement le chlorhydrate de (+)-cis-(2S,3S)-acétyloxy-3 chloro-9 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4.

Point de fusion : 146°C $[\alpha]_D^{20}$ = +20,9° (c = 1 ; MeOH).

Le tableau suivant illustre les structures et propriétés physiques de quelques composés selon l'invention.

Tableau

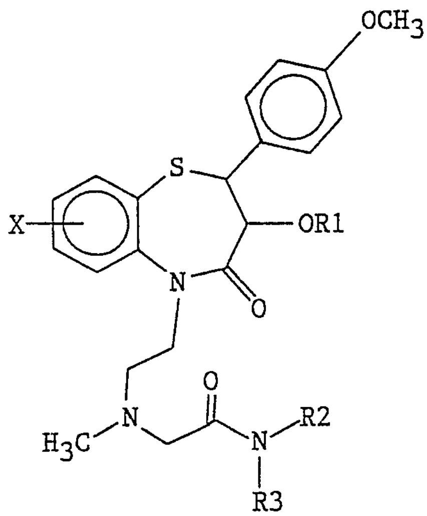

(I)

| N° | X | R1 | R2 | R3 | Sel | $[\alpha]_D^{20}$ (°) c(%), MeOH | F(°C) |
|---|---|---|---|---|---|---|---|
| 1 | H | $COCH_3$ | H | H | ox. | +96,6 (0,5) | 188 |
| 2 | H | $COCH_3$ | H | $iC_3H_7$ | ox. | +90 (0,5) | 119 |
| 3 | H | $COCH_3$ | H | $iC_4H_9$ | ox. | +82,8 (0,5) | 98 |
| 4 | H | $COCH_3$ | H | $tC_4H_9$ | ox. | +79,4 (0,5) | 120 |
| 5 | H | $COCH_3$ | H | $CH_2C_6H_5$ | ox. | +82,2 (0,5) | 185 |
| 6 | H | $COCH_3$ | $CH_3$ | $iC_3H_7$ | ox. | +79,9 (0,5) | 124 |
| 7 | H | $COCH_3$ | $CH_3$ | $C_6H_5$ | ox. | +79,3 (0,5) | 127 |
| 8 | H | $COCH_3$ | $CH_3$ | $CH_2C_6H_5$ | ox. | +73,1 (0,5) | 96 |

| N° | X | R1 | R2 | R3 | Sel | $[\alpha]_D^{20}$ (°) c(%), MeOH | F(°C) |
|---|---|---|---|---|---|---|---|
| 9 | H | $COCH_3$ | $CH_3$ | $CH_2CH_2C_6H_5$ | ox. | +75,2 (0,5) | 95 |
| 10 | H | $COCH_3$ | $C_2H_5$ | $C_2H_5$ | ox. | +81,8 (0,5) | 126 |
| 11 | H | $COCH_3$ | $cC_3H_5$ | $C_6H_5$ | ox. | +80,6 (0,5) | 107 |
| 12 | H | $COCH_3$ | $iC_4H_9$ | $iC_4H_9$ | mal. | +78,1 (0,5) | 112 |
| 13 | H | $COCH_3$ | | 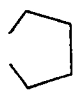 | ox. | +81,2 (0,5) | 148 |
| 14 | H | $COCH_3$ | | 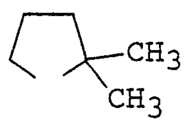 | ox. | +76,6 (0,5) | 110 |
| 15 | H | $COCH_3$ | | 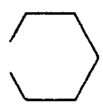 | ox. | +91,6 (0,5) | 145 |
| 16 | H | $COCH_3$ | | 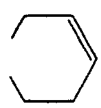 | ox. | +107,1 (0,5) | 152 |
| 17 | H | $COCH_3$ | | 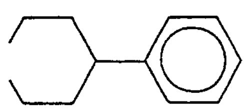 | ox. | +69,1 (0,5) | 167 |
| 18 | H | $COCH_3$ | | 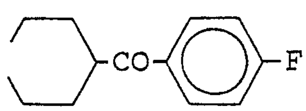 | ox. | +68,5 (0,5) | 164 |
| 19 | H | $COCH_3$ | | 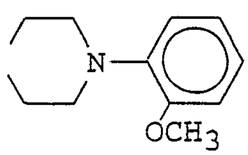 | diox. | +60,9 (0,5) | 128 |
| 20 | H | H | | 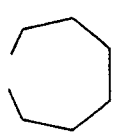 | ox. | +82,3 (0,5) | 102 |

| N° | X | R1 | R2 | R3 | Sel | $[\alpha]_D^{20}$ (°) c(%), MeOH | F(°C) |
|---|---|---|---|---|---|---|---|
| 21 | H | $COCH_3$ | | 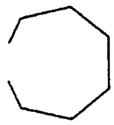 | ox. | +86,11 (0,5) | 147-149 |
| 22 | H | $COCH_3$ | | 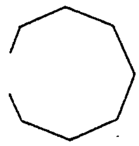 | ox. | +84,7 (0,5) | 129 |
| 23 | H | $COCH_3$ | | 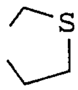 | ox. | +74,9 (0,5) | 145 |
| 24 | H | $COCH_3$ | | 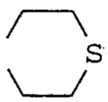 | ox. | +88,1 (0,5) | 123 |
| 25 | H | $COCH_3$ | | 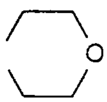 | ox. | +79,9 (0,5) | 117 |
| 26 | H | $COCH_3$ | | 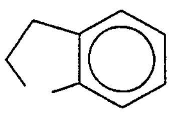 | ox. | +84,4 (0,5) | 139 |
| 27 | H | H | | 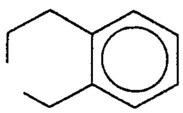 | ox. | +86,7 (0,5) | 118 |
| 28 | H | $COCH_3$ | | 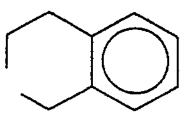 | ox. | +77,5 (0,5) | 144 |
| 29 | H | $COCH_3$ | | 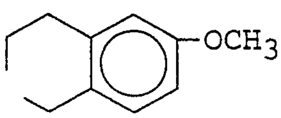 | ox. | +73,3 (0,5) | 142 |
| 30 | H | $COCH_3$ | | 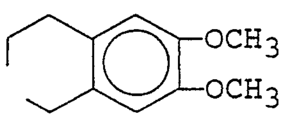 | ox. | +68,7 (0,5) | 115 |
| 31 | H | $COCH_3$ | | 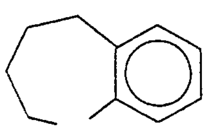 | ox. | +66,9 (0,5) | 140 |
| 32 | 9-Cl | H | | 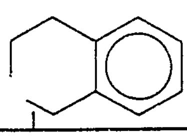 | HCl | 0 | 164 |

| N° | X | R1 | R2 | R3 | Sel | $[\alpha]_D^{20}$ (°) c(%), MeOH | F(°C) |
|---|---|---|---|---|---|---|---|
| 33 | 9-Cl | H | | 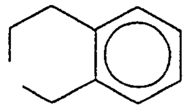 | HCl | +20,3 (1,0) | 162 |
| 34 | 9-Cl | $COCH_3$ | | 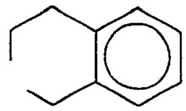 | HCl | 0 | 148 |
| 35 | 9-Cl | $COCH_3$ | | 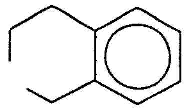 | HCl | +20,9 (1,0) | 146 |
| 36 | 9-Cl | H | | 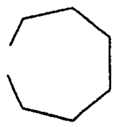 | ox. | 0 | 118 |
| 37 | 9-Cl | H | | 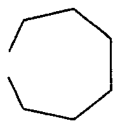 | ox. | +21 (1,0) | 108 |
| 38 | 9-Cl | $COCH_3$ | | 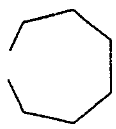 | HCl | 0 | 148 |
| 39 | 9-Cl | $COCH_3$ | | 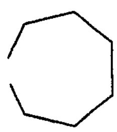 | HCl | +21,1 (1,0) | 126 |

Légende

$iC_3H_7$ : isopropyle
$cC_3H_5$ : cyclopropyle
$iC_4H_9$ : isobutyle
$tC_4H_9$ : tertiobutyle
$C_6H_5$ : phényle
$CH_2C_6H_5$ : benzyle
$CH_2CH_2C_6H_5$ : phénéthyle

HCl : chlorhydrate
ox. : oxalate
diox. : dioxalate
mal. : maléate

Les composés de l'invention ont été soumis à des essais pharmacologiques montrant leur activité comme antagonistes du calcium.

Le protocole expérimental utilisé est une variante de celui utilisé par Godfraind et Kaba (1969), (Blockade or reversal of the contraction induced by calcium and adrenaline in depolarized arterial smooth muscle, Br. J. Pharmac., 36, 549-560).

Les expériences ont été réalisées sur des tronçons d'aorte thoracique de lapin. Les animaux, des "Fauves de Bourgogne" d'un poids moyen de 1,5 kg, sont sacrifiés par dislocation cervicale et exsanguination. L'aorte thoracique est rapidement prélevée et placée dans un milieu de Krebs bicarbonaté oxygéné (95 % $O_2$ + 5 % $CO_2$).

Des tronçons d'aorte de 1 cm de long environ sont préparés et installés dans des cuves à organes de 20 ml contenant de la solution de Krebs bicarbonatée oxygénée (pH 7,4) à 37°C. Deux crochets métalliques en "U" de la longueur des tronçons sont introduits dans la lumière de ceux-ci. L'un des crochets est fixé à la base de la cuve. L'autre, relié à une jauge de contrainte isométrique (Grass FT03), permet l'enregistrement, par l'intermédiaire d'un préamplificateur continu (Grass 7P1) des réponses contractiles des tronçons d'aorte sur un oscillographe à encre (Grass 79B). Cette méthode présente, par rapport aux préparations en spirale ou en anneaux, l'avantage de mieux respecter l'intégrité structurale des vaisseaux et de n'enregistrer que la composante radiale des réponses contractiles qui représente le phénomène intéressant du point de vue fonctionnel (régulation de la pression artérielle). Une tension initiale de 4 g est imposée aux préparations.

De la phénoxybenzamine (1 µM) et du propranolol (1 µM) sont ajoutés aux différents milieux de Krebs afin de supprimer les réponses contractiles liées à l'activation des récepteurs α-et β-adrénergiques vasculaires.

Après une heure de stabilisation dans le milieu de Krebs bicarbonaté, la tension imposée aux aortes est ramenée à 2 g.

Après une période d'attente de 30 minutes, les préparations sont incubées pendant une dizaine de minutes dans une solu tion de Krebs bicarbonatée sans calcium en présence d'EDTA (200 µM) et de propranolol (1 µM). Cette solution est alors remplacée par un milieu de Krebs dépolarisant (riche en potassium et appauvri en sodium) sans calcium et contenant du propranolol (1 µM). Après 5 minutes, une concentration unique de 1 mM de calcium est ajoutée à cette solution et une période de stabilisation de 30 minutes est respectée qui permet aux préparations d'atteindre une contraction stable.

Ensuite, les composés à tester sont ajoutés au bain, à doses cumulatives, un délai de 30 minutes (temps généralement nécessaire pour l'obtention d'un palier) étant respecté entre deux concentrations, jusqu'à disparition totale de la contraction provoquée par 1 mM de calcium ou bien jusqu'à la concentration maximale de 30 µM de produit. En fin d'expérience, une concentration supramaximale de papavérine (300 µM) est ajoutée afin de déterminer la décontraction maximale possible de chaque préparation.

Les valeurs absolues (en grammes) de la contraction initiale après 1 mM de $CaCl_2$ et de la contraction après les différentes concentrations cumulatives de composés vasodilateurs sont obtenues, pour chaque préparation, par différence avec la contraction minimale observée 30 minutes après l'addition finale de 300 µM de papavérine. Le pourcentage de diminution de la contraction, par rapport à la contraction provoquée par 1 mM de calcium, est calculé pour chaque concentration de composé et chaque préparation et ce pourcentage individuel de décontraction est moyenné X ± S.E.M. Les valeurs moyennes obtenues (pondérées par l'inverse de l'erreur standard à la moyenne), sont analysées à l'aide d'un modèle mathématique de courbe sigmoïde. On calcule la concentration molaire provoquant 50% de décontraction de la réponse au calcium ($CE_{50}$), ou bien son antilogarithme ($pCE_{50}$).

Pour les composés de l'invention les $pCE_{50}$ sont de l'ordre de 4,5 à 7.

Les composés de l'invention ont également fait l'objet d'un essai d'inhibition de la liaison spécifique du "PAF", facteur d'activation des plaquettes.

Les animaux sont des lapins d'un poids de 2,5 à 3 kg, anes thésiés au pentobarbital sodique (0,25 mg/kg par voie intraveineuse) et maintenus sous respiration artificielle par intubation. On prélève le sang dans l'artère carotide et on le recueille dans des tubes contenant un anticoagulant à base de citrate. On soumet les tubes à une centrifugation à 100g pendant 15 mn, et on dilue le plasma riche en plaquettes avec 2 volumes de tampon à 10 mM de Tris-HCl, pH 7,5, contenant 150 mM de chlorure de sodium et 2 mM d'EDTA (tampon A). Après centrifugation à 1000g pendant 10 mn à 4°C, on lave le culot avec 2 volumes de tampon A, et on effectue une nouvelle centrifugation.

On met le culot riche en plaquettes en suspension dans un tampon glacé (10 mM de Tris-HCl, pH 7,0, contenant 5 mM de chlorure de magnésium et 2 mM d'EDTA), on l'homogénéise et on le centrifuge à 30000g et à 4°C pendant 10 mn. On répète cette opération, on recueille le culot résultant dans le même tampon, on l'homogénéise et on congèle la suspension de membranes dans l'azote liquide. Pour l'essai d'inhibition de la liaison, on dègele la suspension et on la dilue avec du tampon pour obtenir une teneur d'environ 150 µg de protéine par millilitre.

On fait incuber des quantités aliquotes (30 µg de protéine) de membranes en présence de tampon à 10 mM de Tris-HCl, pH 7,0, contenant 10 mM de chlorure de magnésium et 0,25% (en poids par volume) d'albumine de sérum bovin, pendant 2 h à 25°C, avec 1 nM de PAF tritié ([$^3$H] 1-O-hexadécyl-2-acétyl-sn-glycéryl-3-phosphorylcholine), dans un volume final de 1 ml. On termine l'incubation en recueillant les membranes et en les lavant rapidement avec du tampon glacé sur des filtres Whatman, en utilisant un collecteur de cellules Skatron connecté à une pompe à vide. On sèche les filtres et on les dose par spectrométrie scintigraphique. On définit la liaison spécifique par la différence de radioactivité des membranes observée en l'absence et en la présence

de 1 µM de PAF tritié.
Les essais d'inhibition de la liaison sont effectués dans les conditions décrites, en présence de différentes concentrations de composés à tester. On détermine ensuite la concentration $CI_{50}$ pour chaque composé (concentration qui inhibe de 50% la liaison spécifique) en traçant la courbe d'inhibition selon la méthode des moindres carrés.
Les $CI_{50}$ des composés de l'invention, dans cet essai, se situent entre 1 et 6 µM.

Enfin, les composés de l'invention ont fait l'objet d'un essai d'inhibition de l'agrégation des plaquettes du sang induite par le "PAF-acéther" (1-O-($C_{16}$-$C_{18}$-alkyl)-2-acétyl-sn-glycéryl-3-phosphorylcholine).
L'essai est effectué selon la méthode de Born (J. Physiol., (1963), 168, 178-195) sur des plaquettes de lapin. On prélève le sang par ponction cardiaque, et on le recueille sur du citrate trisodique à 3,8% dans les proportions de 1 volume de solution anti-coagulante pour 9 volumes de sang. On effectue ensuite une centrifugation à 250g pendant 10 mn, on prélève le plasma riche en plaquettes et on procède à la numération des plaquettes.
On soumet le culot à une nouvelle centrifugation, à 3600g pendant 15 mn, de façon à obtenir du plasma pauvre en plaquettes, et on dilue le plasma riche en plaquettes au moyen de plasma pauvre en plaquettes, pour obtenir une suspension contenant de 200000 à 300000 plaquettes par microlitre.
On provoque l'agrégation in vitro au moyen de PAF-acéther à la concentration maximale nécessaire pour obtenir une réponse réversible maximale (normalement entre 1,5 et 3,3 ng/ml). On enregistre les variations de densité optique au moyen d'un agrégomètre (300 µl de plasma riche en plaquettes par cuve, 1100 t/mn à 37°C) jusqu'au dépassement de l'agrégation maximale.
Pour les essais, on dissout les composés de l'invention dans du diméthylsulfoxyde et on les fait incuber pendant 2 mn à 37°C avant l'addition du PAF-acéther.
L'action anti-agrégante des composés s'exprime par la concentration $CI_{50}$, concentration qui inhibe de 50% l'agrégation provoquée par le PAF-acéther.
Les $CI_{50}$ des composés de l'invention, dans cet essai, se situent entre 4 et 8 µM.

Les résultats des essais montrent que les composés de l'in vention sont des antagonistes du calcium et peuvent, à ce titre, être utilisés pour le traitement de diverses affections pour lesquelles ce type d'agents est indiqué.
C'est ainsi qu'en particulier ils peuvent être utilisés en médecine cardiovasculaire pour le traitement d'affections nécessitant des modulateurs des mouvements transmembranaires et intracellulaires du calcium, tout spécialement l'hypertension, l'angor et l'arythmie cardiaque.
Ils sont, en outre, susceptibles de présenter des effets antiathérogènes, antiagrégants plaquettaires, anti-ischémiques cardiaques, anti-ischémiques cérébraux, antimigraineux, antiépileptiques, antiasthmatiques et antiulcéreux.
Dans le domaine cardiovasculaire ils peuvent être utilisés seuls ou associés à d'autres substances actives connues telles que les diurétiques, les β-bloquants, les inhibiteurs de l'enzyme de conversion de l'angiotensine, les antagonistes des récepteurs $\alpha_1$.
Combinés avec des agents destinés à potentialiser leurs effets ou à diminuer leur toxicité, ils peuvent être également indiqués pour le traitement du cancer ou dans les transplantations.

Les composés de l'invention peuvent être présentés sous toutes formes appropriées à l'administration orale ou parentérale, en association avec des excipients connus, par exemple sous forme de comprimés, gélules, dragées, capsules, solutions ou suspensions buvables ou injectables.
La posologie journalière peut aller par exemple de 30 à 300 mg par la voie orale et de 25 à 100 mg par voie parentérale.

**Revendications**

1. Composés, sous forme de diastéréoiosomères purs ou de leurs mélanges, répondant à la formule générale (I)

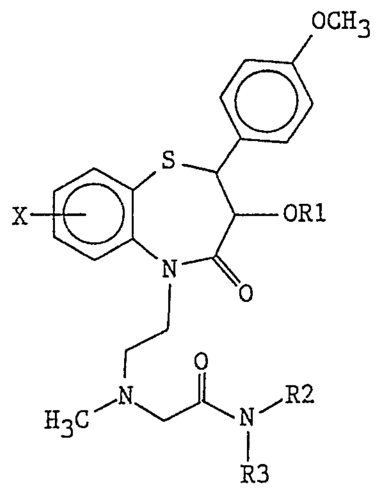

(I)

dans laquelle
R1 représente un atome d'hydrogène ou un groupe alcanoyle en $C_2$-$C_4$,
R2 et R3, indépendamment l'un de l'autre, représentent chacun un atome d'hydrogène, un groupe alkyle en $C_1$-$C_4$ linéaire ou ramifié, un groupe cycloalkyle en $C_3$-$C_5$, un groupe phényle, un groupe benzyle ou un groupe phénéthyle, ou bien encore R2 et R3 forment ensemble, et avec l'atome d'azote qui les porte, un hétérocycle choisi parmi les groupes pyrrolidinyle, diméthyl-2,2 pyrrolidinyle, pipéridinyle, phényl-4 pipéridinyle, (méthoxy-3 phényl)-4 pipéridinyle, (fluoro-4 benzoyl)-4 pipéridinyle, tétrahydro-1,2,3,6 pyridinyle, perhydroazépinyle, perhydroazocinyle, thiazolidine-1,3 yle, thiomorpholinyle, morpholinyle, dihydro-2,3 indolyle, tétrahydro-1,2,3,4 isoquinoléinyle, méthoxy-6 tétrahydro-1,2,3,4 isoquinoléinyle, diméthoxy-6,7 tétrahydro-1,2,3,4 isoquinoléinyle ou tétrahydro-2,3,4,5 1H-benzo[b]azépinyle, et
X représente un atome d'hydrogène ou de chlore,
ainsi que leurs sels d'addition à des acides acceptables en pharmacologie.

2. Composés selon la revendication 1, caractérisés en ce que R3 représente un groupe benzyle.

3. Composé selon la revendication 2, en l'espèce la acétyloxy-3 (méthoxy-4 phényl)-2 [[N-[oxo-2 [N'-(phénylméthyl)méthylamino]-2 éthyl]méthylamino]-2 éthyl]-5 dihydro-2,3 5H-benzothiazépine-1,5 one-4).

4. Composés selon la revendication 1, caractérisés en ce que R2 et R3 représentent, avec l'atome d'azote qui les porte, un groupe phényl-4 pipéridinyle.

5. Composé selon la revendication 4, en l'espèce la acétyloxy-3 (méthoxy-4 phényl)-2 [[N-oxo-2 (phényl-4 pipéridinyl-1)-2 éthyl]méthylamino]-2 éthyl]-5 dihydro-2,3 5H-benzothiazépine-1,5 one-4).

6. Composés selon la revendication 1, caractérisés en ce que R2 et R3 représentent, avec l'atome d'azote qui les porte, un groupe perhydroazépinyle.

7. Composés selon la revendication 6, en l'espèce la acétyloxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4, la chloro-9 hydroxy-3 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4 et la acétyloxy-3 chloro-9 (méthoxy-4 phényl)-2 [[N-[oxo-2 (perhydroazépinyl-1)-2 éthyl]méthylamino]-2 éthyl]-5 dihydro-2,3 5H-benzothiazépine-1,5 one-4).

8. Composés selon la revendication 1, caractérisés en ce que R2 et R3 représentent, avec l'atome d'azote qui les porte, un groupe tétrahydro-1,2,3,4 isoquinoléinyle éventuellement substitué.

9. Composés selon la revendication 8, en l'espèce la acétyloxy-3 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4, la acétyloxy-3 (méthoxy-4 phényl)-2 [[N-[(méthoxy-6 tétrahydro-1,2,3,4 isoquinoléinyl-2)-2 oxo-2 éthyl]méthylamino]-2 éthyl]-5 dihydro-2,3 5H-benzothiazépine-1,5 one-4, la chloro-9 hydroxy-3 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4 et la acétyloxy-3 chloro-9 (méthoxy-4 phényl)-2 [[N-[(tétrahydro-1,2,3,4 isoquinoléinyl-2)-2 oxo-2 éthyl]méthylamino]-2 éthyl]-5 dihydro-2,3 5H-benzothiazépine-1,5 one-4.

10. Composés selon l'une quelconque des revendications précédentes, caractérisés en ce qu'ils sont de configuration (+)-cis (2S,3S).

11. Procédé de préparation des composés selon la revendication 1, caractérisé en ce qu'on fait réagir une benzothiazépinone de formule (V)

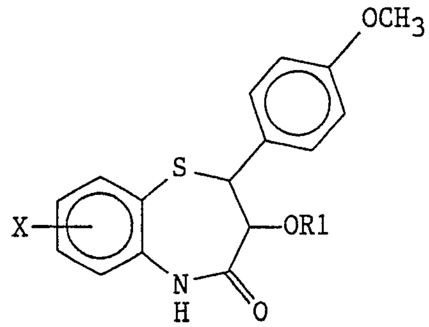

(V)

(dans laquelle R1 et X sont tels que définis dans la revendication 1) avec une amine halogénée de formule (IV)

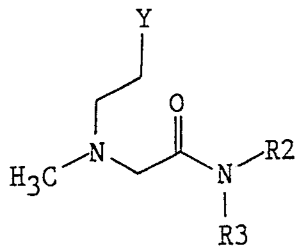

(IV)

(dans laquelle R2 et R3 sont tels que définis dans la revendication 1 et Y représente un atome d'halogène).

12. Composition pharmaceutique, caractérisée en ce qu'elle contient un composé selon l'une quelconque des revendications 1 à 10, associé à un excipient pharmaceutique.

**Revendications pour les Etats contractants suivants: ES, GR**

1. Procédé de préparation de composés, sous forme de diastéréoiosomères purs ou de leurs mélanges, répondant à la formule générale (I)

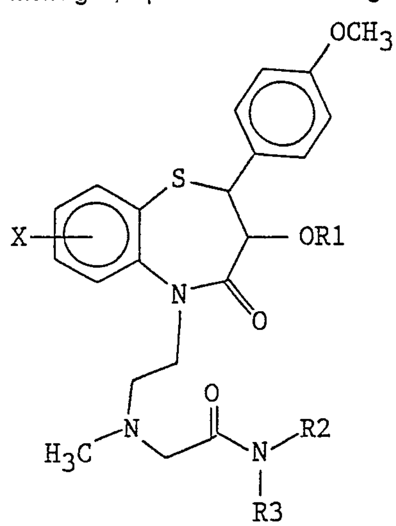

(I)

dans laquelle
R1 représente un atome d'hydrogène ou un groupe alcanoyle en $C_2$-$C_4$,
R2 et R3, indépendamment l'un de l'autre, représentent chacun un atome d'hydrogène, un groupe alkyle en $C_1$-$C_4$ linéaire ou ramifié, un groupe cycloalkyle en $C_3$-$C_5$, un groupe phényle, un groupe benzyle ou un groupe phénéthyle, ou bien encore R2 et R3 forment ensemble, et avec l'atome d'azote qui les porte, un hétérocycle choisi parmi les groupes pyrrolidinyle, diméthyl-2,2 pyrrolidinyle, pipéridinyle, phényl-4 pipéridinyle, (méthoxy-3 phényl)-4 pipéridinyle, (fluoro-4 benzoyl)-4 pipéridinyle, tétrahydro-1,2,3,6 pyridinyle, perhydroazépinyle, perhydroazocinyle, thiazolidine-1,3 yle, thiomorpholinyle, morpholinyle, dihydro-2,3 indolyle, tétrahydro-1,2,3,4 isoquinoléinyle, méthoxy-6 tétrahydro-1,2,3,4 isoquinoléinyle, diméthoxy-6,7 tétrahydro-1,2,3,4 isoquinoléinyle ou tétrahydro-2,3,4,5 1H-benzo[b]azépinyle, et
X représente un atome d'hydrogène ou de chlore,
procédé caractérisé en ce qu'on fait réagir une benzothiazépinone de formule (V)

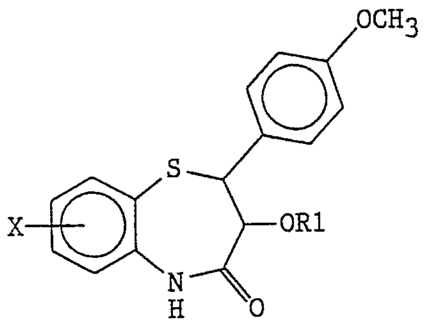

(V)

(dans laquelle R1 et X sont tels que définis ci-dessus) avec une amine halogénée de formule (IV)

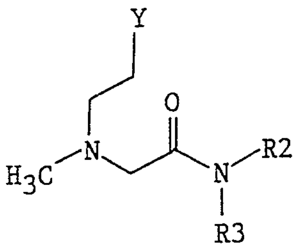

(IV)

(dans laquelle R2 et R3 sont tels que définis ci-dessus et Y représente un atome d'halogène).

2. Procédé selon la revendication 1, caractérisés en ce que R3 représente un groupe benzyle.

3. Procédé selon la revendication 1, caractérisé en ce que R2 et R3 représentent, avec l'atome d'azote qui les porte, un groupe phényl-4 pipéridinyle.

4. Procédé selon la revendication 1, caractérisé en ce que R2 et R3 représentent, avec l'atome d'azote qui les porte, un groupe perhydroazépinyle.

5. Procédé selon la revendication 1, caractérisé en ce que R2 et R3 représentent, avec l'atome d'azote qui les porte, un groupe tétrahydro-1,2,3,4 isoquinoléinyle éventuellement substitué.

Office européen des brevets

# RAPPORT DE RECHERCHE EUROPEENNE

Numero de la demande

EP 89 40 1009

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 256 888 (McNEILAB)<br>* En entier *<br>--- | 1-12 | C 07 D 281/10<br>C 07 D 417/12<br>A 61 K 31/55 |
| Y | FR-A-2 110 275 (HOFFMANN-LA ROCHE)<br>* En entier *<br>----- | 1-12 | |
| | | | **DOMAINES TECHNIQUES RECHERCHES (Int. Cl.4)** |
| | | | C 07 D 281/00<br>C 07 D 417/00 |

Le présent rapport a été établi pour toutes les revendications

| Lieu de la recherche | Date d'achèvement de la recherche | Examinateur |
|---|---|---|
| LA HAYE | 04-07-1989 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)